# EUROPÄISCHE PATENTSCHRIFT

(11) **EP 2 611 398 B1**
(45) Veröffentlichungstag und Bekanntmachung des Hinweises auf die Patenterteilung: **25.02.2015**
(21) Anmeldenummer: 11758383.1
(22) Anmeldetag: 29.08.2011
(51) Int. Cl.: A61F 5/56

(54) **SAUGUTENSIL ZUM VERHINDERN DES SCHNARCHENS UND ANDERER GEWOHNHEITEN**
TEAT DEVICE FOR PREVENTING SNORING AND OTHER HABITS
APPAREIL D'ASPIRATION POUR EMPÊCHER LE RONFLEMENT ET D'AUTRES HABITUDES NÉFASTES

(30) Priorität: 30.08.2010 DE 202010011965 U
(43) Veröffentlichungstag der Anmeldung: 10.07.2013
(73) Patentinhaber: Von Treuenfels, Hubertus, 23701 Eutin (DE)
(72) Erfinder: Von Treuenfels, Hubertus, 23701 Eutin (DE)
(74) Vertreter: Hauck Patentanwaltspartnerschaft mbB
(86) Internationale Anmeldenummer: PCT/EP2011/004325
(87) Internationale Veröffentlichungsnummer: WO 2012/031702

(56) Entgegenhaltungen:
- WO-A1-00/66224
- DE-A1- 3 247 074
- DE-U1- 9 415 655
- US-A- 5 447 168
- US-B1- 6 675 804

## Beschreibung

Die Erfindung bezieht sich auf ein Saugutensil zum Verhindern von Gewohnheiten wie Schnarchen, Mundatmung, Lutschen, Knirschen und Pressen der Zähne und zum Trainieren oder Therapieren oraler Funktionen.

Als Schnarchen bezeichnet man atemabhängige Geräusche, die während des Schlafs durch Flatterbewegungen des erschlafften Gaumensegels entstehen und/oder durch das Zurücksinken der Zunge und des Unterkiefers hervorgerufen werden. Sie gehen deshalb meistens mit offener Mundhaltung und Mundatmung einher. Das Schnarchen ist häufig abhängig von der Lage des Schlafenden. Es kann auch bei behinderter Nasenatmung auftreten und durch Alkoholgenuss, Übergewicht, Überforderung u.a. gefördert werden.

Bekannt ist, dass herkömmliche Beruhigungssauger (Schnuller) geeignet sind, das Schnarchen in vielen Fällen zu unterbinden. Beruhigungssauger haben einen starren Mundschild, an dem ein Saugkörper aus weichelastischem Material befestigt ist. Der Mundschild ist ein Formkörper aus einem Kunststoff und der Saugkörper besteht aus Latex oder Silikon. Bei der Benutzung ist der Mundschild vor den Lippen des Trägers angeordnet, um ein Herunterschlucken des Beruhigungssaugers zu verhindern. Die Anordnung des Mundschildes vor den Lippen des Trägers wird unter Umständen als störend empfunden.

Ferner gibt es schon Kiefer-Protrusionsschienen, die das Zurücksinken des Unterkiefers im Schlaf verhindern und ihn in einer korrekten Lage halten, sodass die Zunge nicht zurückfallen und die Atemgeräusche fördern oder gar die Atemwege blockieren kann.

Zudem ist eine Vorhofplatte oder Vorhofschiene bekannt, die in den Mundvorhof, den Raum zwischen Lippen und Frontzähnen gesetzt wird und somit die Mundatmung blockiert. Konturen auf der Innenseite der Vorhofschiene/Vorhofplatte dienen als Spielzeug für die Zunge. Durch den ausgelösten Schluckreflex wird die Zunge gegen die Frontzähne und die Vorhofschiene/Vorhofplatte gepresst und dort durch den entstehenden Unterdruck platziert. Der Freiraum der Luftröhre wird erweitert und die Vibration des Gaumensegels unterbunden. Die Schnarchgeräusche werden somit verhindert. Das bioplastische Material wird durch die Erwärmung im Mund noch elastischer und passt sich individuell dem Mundvorhof an.

Die US-A-6 675 804 beschreibt ein Saugutensil mit einer mehrere Lappen aufweisenden Struktur zum Aufnahmen der Zunge, einem wellenförmigen Verbinder zum Verbinden der genannten Struktur mit einer inneren Lippenplatte und einem hohlen Röhrchen, welches die Lippenplatte mit einer kuppelförmigen Struktur auf einem äußeren Mundschild verbindet. Die Vorrichtung wird in eine Anwendungsstellung in die Mundhöhle eingesetzt, sodass eine Bewegung der Zunge durch die mehrere Lappen aufweisende Struktur eingeschränkt wird und die Zähne auf den wellenförmigen Verbinder beißen, wobei die Lippenplatte zwischen den Zähnen und den inneren Teilen der oberen und unteren Lippen angeordnet ist. Die Vorrichtung zielt darauf ab, die Atmung während der Nacht zu normalisieren. Durch die Verbindung einer Öffnung in der Lippenplatte mit der Atmosphäre soll die Mundatmung gefördert werden.

Davon ausgehend liegt der Erfindung die Aufgabe zugrunde, eine verbesserte Vorrichtung zum Verhindern von Gewohnheiten wie Schnarchen, Mundatmung, Lutschen, Knirschen und Pressen der Zähne sowie zum Trainieren/Therapieren oraler Funktionen zur Verfügung zu stellen.

Die Aufgabe wird durch ein Saugutensil mit den Merkmalen des Anspruchs 1 gelöst. Vorteilhafte Ausgestaltungen des Saugutensils sind in den Unteransprüchen angegeben.

Das erfindungsgemäße Saugutensil zum Verhindern von Gewohnheiten wie Schnarchen, Mundatmung, Lutschen, Knirschen und Pressen der Zähne und zum Trainieren/Therapieren oraler Funktionen umfasst einen zumindest teilweise aus einem flexiblen Flachmaterial bestehenden, sich an die Oberfläche der Zähne und des Zahnfleisches anpassenden Mundvorhofschild, der so lang ist, dass er seitlich zumindest bis zu den Prämolaren reicht, und einen von der Innenseite des Mundvorhofschildes vorstehenden und an seiner Basis mit dem Mundvorhofschild verbundenen weichelastischen Saugkörper.

Grundlage für die Wirksamkeit des neuen Saugutensils ist der angeborene und lebenswichtige Saugreflex. Bis ins hohe Erwachsenenalter kommt es zu dieser Reaktion, sobald ein angemessener Saugkörper in den Mund gelangt. Schließlich ernährt sich der Mensch saugend-trinkend. Auch die weich- und feuchtgekauten Speisen saugt er sich mit und auf der Zunge zu einem Bolus zusammen, um sie schluckend in den Schlund zu befördern. Der Kontakt der Zunge mit dem Saugkörper bzw. der Mundschluss durch den Mundvorhofschild löst den Saugreflex aus. Im Zuge dieses Saugvorganges kommt es zu einem hinteren Verschluss der Mundhöhle. Mit der Anhebung des Zungenrückens und der Anspannung des Gaumensegels wird der Luftstrom und damit das flatternde Schnarchgeräusch über den Mund unterbunden. Gegenüber dem konventionellen Beruhigungssauger bietet das neue Saugutensil zwei entscheidende Vorzüge. Der äußerlich sichtbare und harte Lippenschild wird ersetzt durch einen unsichtbar hinter den Lippen anzuordnenden Mundvorhofschild, der zumindest teilweise aus einem flexiblen und damit angenehm zu tragenden Flachmaterial besteht. Der Mundvorhofschild passt sich an den Mundvorhof an und führt nicht zu einer von außen leicht erkennbaren Veränderung der Mundpartie. Er kann sehr dünn ausgeführt, ggf. sogar als dünnwandige Membran geformt sein. Der dünne und flache Mundvorhofschild ist schleimhautfreundlich, anschmiegsam und von außen unauffällig. Insbesondere bewirkt er keine vorwölbend entstellende Lippenveränderung und kein schnullerhaftes Aussehen wie bei der herkömmlichen Mundvorhofplatte. So gestaltet sich das Saugutensil attraktiver und ist insbesondere für die Rücksichtnahme in der Partnerschaft geeignet.

Zudem ist es effektiver als ein herkömmlicher Beruhigungssauger, weil dem Schnarchen zusätzlich durch den vorderen Mundschluss entgegengewirkt wird. Diesen sichern und verstärken die Lippen, die den Mundvorhofschild als Reaktion auf seinen Berührungsreiz und zur Gewährleistung eines Saugeffektes umschließen. Der flexible Mundvorhofschild aus Flachmaterial passt sich der Oberfläche der Zähne und des Zahnfleisches an und dichtet im Zusammenwirken mit dem Speichel und der Schleimhaut den Mund nach außen ab. Beim Saugen baut sich infolgedessen ein Unterdruck in der Mundhöhle auf. Sobald die Mundhöhle hinten durch Zungen-Gaumenkontakt abgedichtet und vom durch Lippenkontakt sowie durch Anlage des Mundvorhofschildes auf der Schleimhaut geschlossen wird, entfaltet die Zunge ihre kolbenartigen Bewegungen. Ein rückverlagerter Unterkiefer wird dabei mitsamt der Zunge und der Unterlippe spontan vorgeschoben, um den erforderlichen Mundschluss (Lippenkontakt), die ideale Lagebeziehung beider Kiefer zueinander und den Kontakt zwischen Zunge und vorderem Gaumen zu erreichen. Die geräuschhafte Mundatmung tritt somit zugunsten einer stillen Nasenatmung in den Hintergrund.

Somit beruht die ein Schnarchen verhindernde oder zumindest vermindernde Wirkung des Saugutensils auf einem Doppeleffekt des aktiven hinteren Mundschlusses am Oropharynx (Schlundschluss) und dem aktiven vorderen Mundschluss am Vestibulum. Der Doppeleffekt verhindert Mundatmung einschließlich geräuschhafter Vibration des Gaumensegels durch reaktive Anspannung der Muskeln (Lippen, Zunge, Halsmuskeln, Schlundmuskeln und viele andere).

Das erfindungsgemäße Saugutensil ist grundsätzlich für die Anwendung durch Menschen geeignet, die an obstruktiven schlafbezogenen Atemstörungen (OSAS) leiden und deren Schnarch-Schlafproblem primär mit den strukturellen und funktionellen Defiziten des Oronasalraumes zusammenhängen. Darüberhinaus kann das Saugutensil ergänzend Schlafgestörten mit einer Apnoe helfen, die an anderen bzw. zusätzlichen Beschwerden leiden. Dazu gehören Erkrankungen wie: Alkoholismus, Adipositas, Allergien, Hormonstörungen, Stoffwechselprobleme, Herz-Kreislauferkrankungen u.a. Auch hier könnte das Saugutensil in Verbindung mit HNO-ärztlicher, internistischer, psychologischer, zahnärztlicher u.a. Disziplinen Wirkung zeigen.

Der Mundvorhofschild besteht bevorzugt insgesamt aus einem flexiblen Flachmaterial. Die Erfindung umfasst jedoch auch Ausführungen des Mundvorhofschildes, die nur teilweise aus einem flexiblen Flachmaterial bestehen. Insbesondere umfasst die Erfindung Ausführungen, bei denen der Mundvorhofschild in den Randbereichen aus einem flexiblen Flachmaterial besteht und in einem Zentralbereich aus einem härteren Material. Ferner umfasst sie Mundvorhofschilde, die in den Randbereichen aus einem flexiblen Flachmaterial bestehen und in mehreren inneren Bereichen aus einem härteren Material. Falls der Mundvorhofschild nur teilweise aus einem flexiblen Flachmaterial besteht, kann er einteilig aus einem einzigen Material oder aus mehreren Materialen ausgeführt sein. Bei Herstellung aus nur einem einzigen Material können flexible und harte Bereiche durch unterschiedliche Materialstärken verwirklicht sein. Bei Herstellung aus mehreren Materialen können weiche und harte Bereiche durch Komponenten unterschiedlicher Weichheit bzw. Härte verwirklicht sein.

Das Saugutensil umfasst einen von der Innenseite des Mundvorhofschildes vorstehenden und an seiner Basis mit dem Mundvorhofschild verbundenen weichelastischen Saugkörper. Der Saugkörper löst in Verbindung mit dem übrigen Saugutensil den Saugreflex aus, so dass die oben beschriebenen vorteilhaften Wirkungen besonders gefördert werden. Beim Saugen dehnt sich das Material des weichelastischen Saugkörpers entsprechend der für den Schluckvorgang vorbereiteten Nahrung wiederholt nach hinten. Zudem wird durch die Kontakt von Ober- und Unterkiefer verhindernde Anordnung des Saugkörpers zwischen den Zähnen einem Knirschen und Pressen entgegengewirkt.

Ähnlich wie bei der Anwendung eines Beruhigungssaugers (Schnuller) kann das Saugutensil zur Verhinderung oder Abgewöhnung von Lutschgewohnheiten an Fingern (o. a. Objekten) eingesetzt werden.

Die unter Logopäden u. a. Therapeuten bekannte therapeutische Anwendung von speziellen Saugern zur Verbesserung der Atem-, Saug-, Schluck- und Sprechfunktion kann mit dem erfindungsgemäßen Sagutensil ebenso, wenn nicht gar z. T. effektiver bzw. einfacher durchgeführt werden.

Gemäß einer Ausgestaltung weist der Saugkörper einen durch eine Öffnung in der Außenseite des Mundvorhofschildes zugänglichen Hohlraum auf. Diese besonders nachgiebige Konstruktion fördert den Saug- und Lutscheffekt.

Gemäß einer weiteren Ausgestaltung weist der Saugkörper einen Saugernippel und einen einenends mit dem Saugernippel und einen anderenends mit dem Mundvorhofschild verbundenen Hals auf. Der Saugernippel hat einen im Vergleich zum Hals größeren Querschnitt, wodurch der Saugeffekt verstärkt wird. Der Hals ist aufgrund seines geringeren Querschnittes besser zwischen den Zähnen platzierbar.

Gemäß einer weiteren Ausgestaltung besitzt der Saugkörper eine orthodontische Form. Orthodontische Saugkörper zeichnen sich durch einen Saugernippel mit einer konvexen Wölbung der dem Gaumen zugewandten Oberseite, einer nach hinten und nach oben verlaufenden Anschrägung an der der Zunge zugewandten Unterseite und einer konkaven Wölbung in der Anschrägung aus. Alternativ werden auch Saugkörper mit einem Saugernippel in einer runden Kirschform hergestellt, die dem Konzept der Erfindung aber weniger dienlich sind als Saugkörper mit orthodontischer Form. Orthodontische Saugkörper und Saugkörper mit einem Saugernippel mit einer runden Kirschform sind vorzugsweise distal geschlossen.

Gemäß einer Ausgestaltung weist der distal geschlossene Saugkörper in Richtung seiner Längsachse eine Ausdehnung von 20 bis 35 mm auf. Je nach Alter oder Mundgröße oder Mundform kann der Saugkörper im angegebenen Bereich gewählt werden.

Gemäß einer Ausgestaltung hat der Saugkörper und/oder der Mundvorhofschild mindestens eine napfförmigen Vertiefung. Die napfförmige Vertiefung ist vorzugsweise geschlossen und durch eine Einwölbung einer Wand des Saugkörper und/oder des Mundvorhofschildes gebildet. Die napfförmige Vertiefung wirkt als Saugnapf, der - je nach Positionierung - an der Zunge, am Gaumen, an der Schleimhaut oder am Zahnfleisch anhaften kann. Diese Ausgestaltung wirkt stimulatorisch auf den Saug- und Lutschreflex. Außerdem verbessert und sichert sie die vorgesehene Haftung und Positionierung des Saugutensils einerseits und der Zunge andererseits. Die napfförmige Vertiefung kann insbesondere eine größere Einstülpung oder Mulde in einer Fläche des Saugkörpers und/oder Mundvorhofschildes sein. Im Bereich der napfförmigen Vertiefung ist der Saugkörper und/oder Mundvorhofschild elastisch ausgebildet. Hierbei kann die napfförmige Vertiefung dieselbe Wandstärke wie in den angrenzenden Bereichen des Saugkörpers und/oder des Mundvorhofschildes aufweisen. Ferner kann die napfförmige Vertiefung eine geringere Wandstärke als die angrenzenden Bereiche haben.

Gemäß einer bevorzugten Ausgestaltung weist der Saugkörper die napfförmige Vertiefung in eine Fläche auf, an der beim Gebrauch die Zunge anliegt, und/oder in einer Fläche, an der beim Gebrauch der Gaumen anliegt, und/oder weist der Mundvorhofschild die napfförmige Vertiefung in einer Fläche auf, die beim Gebrauch dem Zahnfleisch und/oder der Lippen-Wangenschleimhaut zugewandt ist. Diese Ausgestaltungen ermöglichen eine Saughaftung zwischen Zunge bzw. Zungenrücken und Saugkörper und/oder zwischen Gaumen und Saugkörper und/oder zwischen Zahnfleisch und/oder Lippen-Wangenschleimhaut und Mundvorhofschild. Das Saugutensil kann mit einer oder mehreren napfförmigen Vertiefungen an einer oder verschiedenen der vorerwähnten Positionen versehen sein.

Gemäß einer Ausgestaltung weist der Saugkörper des Saugutensils eine distale Öffnung auf. Vorzugsweise erweitert sich der Saugkörper zur distalen Öffnung hin. Die Erweiterung des Saugkörpers zur distalen Öffnung ist vorzugsweise kelchförmig oder trompetenförmig. Die distale Öffnung des Saugkörpers dient dazu, die Zungenspitze des Benutzers aufzunehmen. Diese Ausgestaltung wirkt sich ähnlich wie die bereits beschriebene napfförmige Vertiefung stimulatorisch auf den Saug- und Lutschreflex und vorteilhaft für die Haftung und Positionierung des Saugutensils einerseits und der Zunge andererseits aus. Außerdem findet das Saugutensil aufgrund der speziellen Gestaltung des Saugkörpers beim Benutzer eine erhöhte Akzeptanz. Gemäß einer bevorzugten Ausgestaltung ist der Saugkörper glatt und/oder sind die äußeren Ränder der Öffnung gerundet. Gemäß einer weiteren Ausgestaltung weist der Saugkörper einen Hals und nur einen damit verbundenen Abschnitt eines Saugernippels auf, der die distale Öffnung hat. Der Abschnitt des Saugernippels entspricht vorzugsweise einem proximalen Abschnitt eines Saugernippels eines orthodontischen Saugkörpers oder eines Saugkörpers mit Kirschform, dem ein distaler Abschnitt des Saugernippels fehlt.

Gemäß einer weiteren Ausgestaltung ist die Öffnung mittels eines lösbar am Mundvorhofschild gehaltenen Verschlussteils verschlossen. Grundsätzlich bildet sich der Saugeffekt bei dem Saugutensil mit distaler Öffnung des Saugkörpers und Öffnung des Saugkörpers im Mundvorhofschild auch ohne das Verschlussteil, wenn der Benutzer die Lippen geschlossen hat. Falls der Benutzer Schwierigkeiten mit dem Lippenschluss hat, bewirkt das Verschlussteil, dass die Zunge dennoch einen Unterdruck erzeugen kann. Das Verschlussteil ist lösbar am Mundvorhofschild gehalten, sodass es nach Bedarf eingesetzt oder entnommen werden kann. Das Verschlussteil kann beispielsweise wie ein Druckknopf in die Öffnung des Mundvorhofschildes einknöpfbar sein. Das Verschlussteil kann eine Verschlussplatte mit einem druckknopfartigen Vorsprung haben, der druckknopfartig in die Öffnung des Mundvorhofschildes einpressbar ist. Der Mundvorhofschild bzw. der Saugkörper kann eine Rille oder Nut zur Aufnahme eines Wulstes am Vorsprung haben. Die Platte des Verschlussteiles kann sich abdichtend an die Außenseite des Mundvorhofschildes anlegen. Die Platte kann aus einem membranartigen, flexiblen Material bestehen.

Gemäß einer bevorzugten Ausgestaltung hat der Saugkörper mit einer distalen Öffnung in Richtung seiner Längsachse eine Ausdehnung von 15 bis 25 mm.

Gemäß einer weiteren Ausgestaltung hat dieser Saugkörper eine größte Breite von 20 bis 30 mm. Gemäß einer weiteren Ausgestaltung hat der Hals dieses Saugkörpers an seiner schmalsten Stelle eine Breite von 15 bis 25 mm und/oder eine Höhe von 7 bis 10 mm.

Gemäß einer weiteren Ausgestaltung weist die Öffnung im Mundvorhofschild eine Breite von 13 bis 20 mm und/oder eine Höhe von 5 bis 8 mm auf.

Diese Angaben beziehen sich auf die Anordnung des Saugutensils im Mund eines Benutzers, wobei die Breite in horizontaler Richtung und die Höhe in vertikaler Richtung gemessen werden.

Gemäß einer Ausgestaltung enthält der Mundvorhofschild eine an die Anatomie des Mundvorhofs angepasste Kontur. So lässt sich das Saugutensil angenehm tragen und Reizungen der Mundschleimhaut werden vermieden.

Gemäß einer weiteren Ausgestaltung folgt der Mundvorhofschild einer langgestreckten Kontur. Der Mundvorhofschild ist so lang, dass er seitlich zumindest bis zu den Prämolaren reicht.

Gemäß einer weiteren Ausgestaltung erscheint die Kontur des Mundvorhofschildes an den gingivalen Rändern im Nachbarbereich zu den Lippenbändchen tailliert. Durch diese Aussparungen werden Reizungen der Lippenbändchen vermieden. Gemäß einer Ausgestaltung hat der Mundvorhofschild im Bereich der Taille an der Oberseite und/oder an der Unterseite V-förmige Aussparungen.

Gemäß einer weiteren Ausgestaltung zeigt der Mundhofschild eine an den äußeren Rändern in Umfangsrichtung gerundete Kontur. Hierdurch wird eine weitere Anpassung an die Anatomie der Umschlagfalte des Mundvorhofes erreicht. Gemäß einer bevorzugten Ausgestaltung hat der Mundvorhofschild einen Randbereich, der zur Peripherie hin dünn ausläuft. Hierdurch ist er besonders weich und anschmiegsam.

Gemäß einer bevorzugten Ausgestaltung sieht der Mundvorhofschild eine katzenzungenförmige (bzw. löffelbiscuitförmige) Kontur vor. Damit wird ein langgestrecktes Format verwirklicht, das im Nachbarbereich zu den Lippenbändchen tailliert und an den äußeren Rändern gerundet ist.

Gemäß einer Ausgestaltung hat der Mundvorhofschild lateral eine maximale Ausdehnung von 80 bis 140 mm, und/oder an der Taille eine minimale Ausdehnung von 15 bis 35 mm und/oder beidseitig neben der Taille eine maximale Ausdehnung von 25 bis 50 mm. Mit Abmessungen in den genannten Bereichen ist der Mundvorhofschild für Träger unterschiedlicher Alterssparten oder Mundformen oder Mundgrößen geeignet. Ferner kann der Mundvorhofschild durch Zuschneiden individuell angepasst werden.

Ein Mundvorhofschild, der von vielen Anwendern ohne Anpassung getragen werden kann, hat lateral eine maximale Ausdehnung von 115 bis 135 mm und/oder an der Taille eine minimale Ausdehnung von 15 bis 25 mm und/oder beidseitig der Taille eine maximale Ausdehnung von 22,5 bis 37,5 mm.

Der Mundvorhofschild aus flexiblem Flachmaterial kann an den jeweiligen Träger angepasst werden. Hierfür kann der Mundvorhofschild z.B. mittels einer Schere oder eines Messers oder einer Stanze zurechtgeschnitten werden. Das Material des Mundvorhofschildes muss entsprechend gewählt werden.

Gemäß einer Ausgestaltung zeigt der Mundvorhofschild mindestens eine vom Rand des Mundvorhofschildes beabstandete Markierungslinie mit einem der Anatomie des Mundvorhofes und/oder der Kontur des Mundvorhofschildes angepassten Verlauf. Die Markierungslinie kann den Träger beim Anpassen des Mundvorhofschildes unterstützen, indem sie eine Schneidlinie vorgibt, entlang der der Mundvorhofschild gekürzt werden kann. Wenn der Mundvorhofschild eine der Anatomie des Mundvorhofes angepasste Kontur aufweist, kann die Markierungslinie der Kontur des Mundvorhofschildes parallel folgen. Der Mundvorhofschild kann mit mehreren Markierungslinien versehen sein, um eine Anpassung an Mundvorhöfe unterschiedlicher Form und/oder Größe zu ermöglichen. Die Markierungslinie kann eine unterbrochene oder eine ununterbrochene Linie sein.

Gemäß einer Ausgestaltung weist der Mundvorhofschild auf einer Seite mindestens eine Bissleiste auf. Ein Saugutensil mit oder ohne Saugkörper kann mit einer Bissleiste versehen sein. Der Mundvorhofschild kann mit einer einzigen Bissleiste in der Mitte ausgeführt sein, die sich im Gebrauch in der Transversalebene über die Schneidekanten des Trägers hinweg erstreckt. Am Mundvorhofschild mit oder ohne Saugkörper, können auch mehrere, z.B. drei Bissleisten in der Transversalebene angeordnet sein. In diesem Fall sind bevorzugt am Mundvorhofschild je eine Bissleiste auf verschiedenen Seiten der Sagittalebene angeordnet, während sich die dritte Bissleiste in der Mitte befindet. Falls das Saugutensil keinen Saugkörper hat, kann die mittlere Bissleiste in derselben Ebene wie die beiden seitlichen Bissleiste angeordnet sein. Bei einem Saugutensil mit Saugkörper kann sie sich ober- und/oder unterhalb des Saugkörpers befinden.

Der Saugkörper und/oder die Bissleiste kann mittels eines geeigneten Befestigungsmittels mit dem Mundvorhofschild verbunden sein. Bevorzugt ist der Saugkörper und/oder die Bissleiste einteilig mit dem Mundvorhofschild verbunden, beispielsweise durch Verkleben oder Verschweißen. Bevorzugt sind Saugkörper und/oder Bissleiste und Mundvorhofschild von vornherein als ein einziges Teil aus einem zusammenhängenden Material hergestellt.

Gemäß einer weiteren Ausgestaltung besteht der Saugkörper und/oder der Mundvorhofschild und/oder die Bissleiste aus Naturkautschuk (hergestellt aus Latex) und/oder Silikon und/oder thermoplastischem Elastomer und/oder bioplastischem Kunststoff. Der Saugkörper und/oder der Mundvorhofschild und/oder die Bissleiste kann aus einer einzigen Materialkomponente hergestellt sein oder aus mehreren Materialkomponenten bestehen.

Eine oder mehrere Bissleisten können in einer Ausgestaltung getrennt vom Saugutensil aus demselben oder aus einem anderen Material hergestellt und als Ergänzung/Erweiterung mit dem Saugutensil mechanisch verknüpft werden. Dies ist z.B. durch Einknöpfen von mit den Bissleisten verbundenen Bolzen oder Stegen mit endseitigen Verbreiterungen (z. B. Bolzenköpfen oder Halteplatten) in Öffnungen (Löcher oder Schlitze) des Mundvorhofschildes möglich. Mehrere Bissleisten, sei es in der Mitte oder an den Seiten, können in einer weiteren Ausgestaltung unabhängig voneinander oder im Verbund als einteiliges Bissleistenelement getrennt vom Saugutensil und aus einem anderen Material hergestellt und mit dem Saugutensil mechanisch verknüpft werden.

Ferner ist es möglich, den Saugkörper und/oder den Mundvorhofschild und/oder die Bissleiste in besonders beanspruchten Bereichen mit einer Verstärkung zu versehen, beispielsweise einem in den Saugkörper und ggf. in den Mundvorhofschild eingebetteten Verstärkungsstreifen. Die Bissleiste kann eine Aufbissleiste sein, die nicht oder nur unwesentlich verformt wird, wenn der Träger darauf beißt. Ferner kann die Bissleiste als Einbissleiste ausgebildet sein, wobei sie zumindest an den Außenseiten, auf die der Träger beißt, aus einem weichen und/oder plastisch verformbaren Material besteht, in das der Träger hineinbeißen kann. Ferner kann der Saugkörper eine oder mehrere keilförmige Aussparungen (Rille) auf der Innenseite aufweisen, die ihm eine größere Nachgiebigkeit verleihen, beispielsweise wie in der WO 2008154968 A1 beschrieben.

Der Saugkörper und/oder der Mundvorhofschild haben bevorzugt eine Wandstärke von 0,5 bis 3 mm, weiterhin bevorzugt 1 bis 2 mm. Die Ausmaße der Bissleiste weisen bevorzugt eine Wandstärke (Höhe) von 4 bis 8 mm, eine Länge von 20 bis 30 mm und eine Breite von 8 bis 12 mm auf. Die Wandstärken aller Komponenten des Saugutensils (Mundvorhofschild, Saugkörper und Bissleiste) können auch, je nach Ausführung durch die genannten Materialien, abweichend von den angegebenen Maßen, unterschiedlich ausfallen.

Gemäß einer weiteren Ausgestaltung ist das Saugutensil im Tauchverfahren oder im Spritzgießverfahren hergestellt.

Die Erfindung betrifft ferner eine Schablone aus einem Flachmaterial mit an die Anatomie des Mundvorhofes angepasster Kontur und/oder Markierungslinie. Der Benutzer kann zunächst eine Schablone in den Mundvorhof einsetzen und die Kontur des Mundvorhofschildes an die Kontur der Schablone anpassen, wenn die Schablone angenehm zu tragen ist. Falls dies nicht sogleich gegeben ist, kann der Benutzer die Schablone zuschneiden, ggfs. entlang der Markierungslinie, bis sie optimal passt. Danach kann er die Schablone als Schneidhilfe zum Anpassen des Mundvorhofschildes benutzen.

Gemäß einer weiteren Ausgestaltung hat die Schablone eine langgestreckte und/oder taillierte und/oder an den Enden gerundete Kontur und/oder Markierungslinie. Bevorzugt hat die Schablone eine katzenzungenförmige Kontur und/oder Markierungslinie. Weiterhin bevorzugt hat die Schablone verschiedene Markierungslinien, die das Zuschneiden verschiedener Konturen erleichtern.

Die Schablone besteht bevorzugt aus einem inerten und feuchtigkeitsunempfindlichen Material. Bevorzugt besteht die Schablone aus einem Material, das sich möglichst ähnlich wie das Material des Mundvorhofschildes anfühlt. Bevorzugt besteht die Schablone aus demselben Material wie der Mundvorhofschild. Beispielsweise besteht die Schablone aus einem Kunststoff oder einer Kunststofffolie.

Ferner betrifft die Erfindung ein Set mit mehreren Schablonen mit verschiedenen Konturen und/oder mit verschiedenen Markierungslinien. Der Benutzer kann die geeignete Schablone und dann nach Erprobung das dazu passende Saugutensil auswählen. Hierfür kann das Saugutensil mit Mundvorhofschilden verschiedener Größe und/oder mit verschieden geformten Mundvorhofschilden angeboten werden, wobei den verschiedenen Mundvorhofschilden jeweils eine Schablone des Sets zugeordnet ist. Die Zuordnung kann dem Benutzer über eine übereinstimmende Kennzeichnung des Saugutensils und der zugeordneten Schablone erleichtert werden.

Schließlich betrifft die Erfindung ein Set mit einem erfindungsgemäßen Saugutensil und mindestens einer erfindungsgemäßen Schablone. Der Benutzer kann die geeignete Schablone auswählen bzw. passend zuschneiden und die Kontur der Schablone auf den Mundvorhofschild des Saugutensils übertragen.

Das erfindungsgemäße Saugutensil kann je nach Indikation (Knirschen, Mundatmung mit oder ohne Schnarchen usw.) und nach Akzeptanz beim Träger ausgestaltet sein. So kann das Saugutensil mit oder ohne Saugkörper, mit oder ohne Bissleisten, mit oder ohne Saugnäpfen, mit Saugnäpfen ausschließlich am Saugkörper, ausschließlich am Mundvorhofschild oder am Saugkörper und Mundvorhofschild, mit oder ohne Markierungslinien und mit oder ohne Schablone ausgeführt sein. Kombinationen sind möglich und stehen dem Benutzer bzw. dem behandelnden Arzt als Optionen zur Verfügung.

Die Erfindung wird nachfolgend anhand der anliegenden Zeichnungen von Ausführungsbeispielen näher erläutert. In den Zeichnungen zeigen:

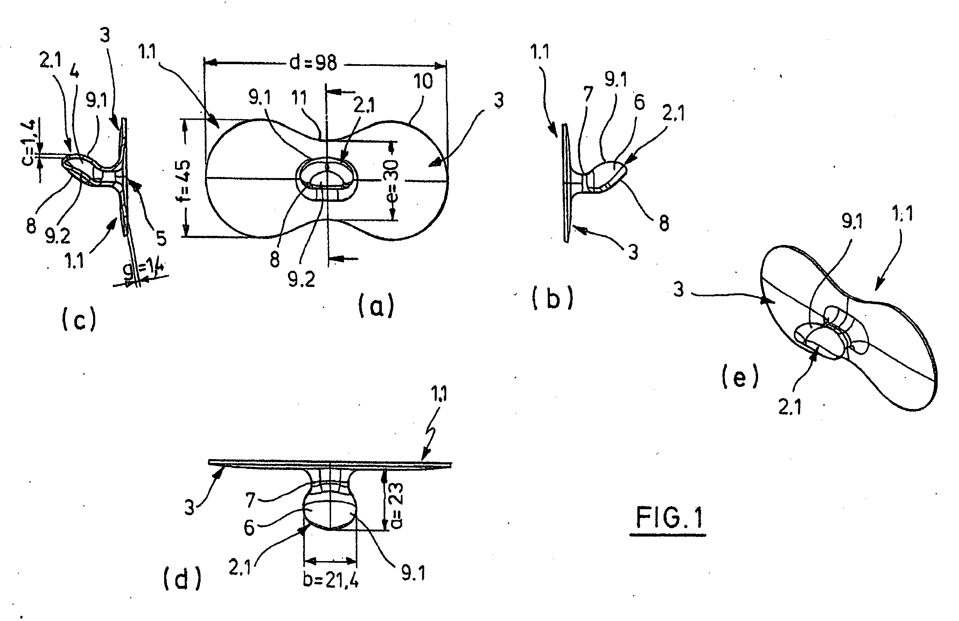

- Fig. 1 a bis e: ein Saugutensil für ein Kind oder einen Träger mit einer kleinen Mundgröße in Vorderansicht (Fig. 1a), einer Ansicht von der linken Seite (Fig. 1b), einem Vertikalschnitt von rechts (Fig. 1c), einer Draufsicht (Fig. 1d) und in einer Perspektivansicht schräg von oben und von der rechten Seite (Fig. 1e).

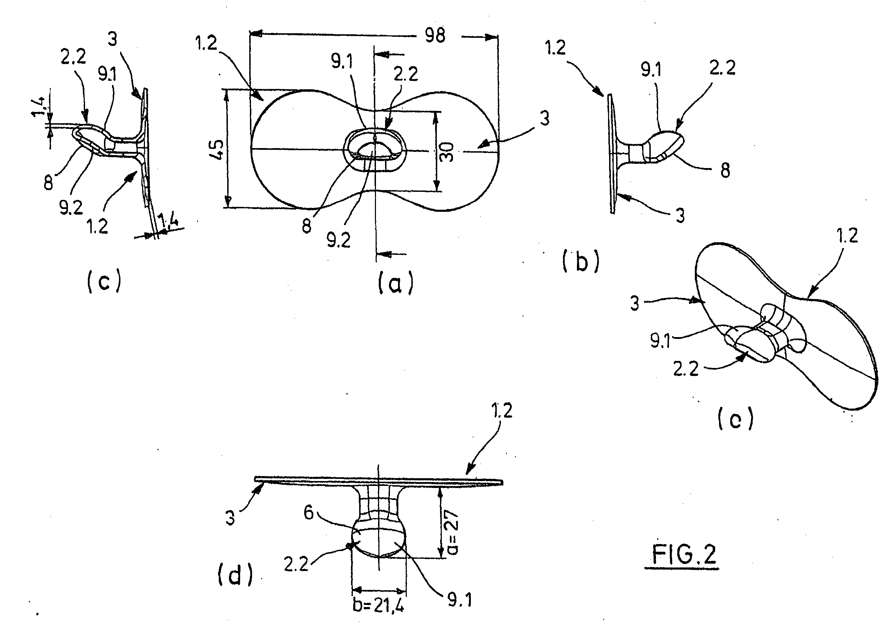

- Fig. 2 a bis e: ein Saugutensil für einen Jugendlichen oder einen Träger mit einer mittleren Mundgröße in Vorderansicht (Fig. 2a), einer Ansicht von der linken Seite (Fig. 2b), einem Vertikalschnitt von rechts (Fig. 2c), einer Draufsicht (Fig. 2d) und in einer Perspektivansicht schräg von oben und von der rechten Seite (Fig. 2e).

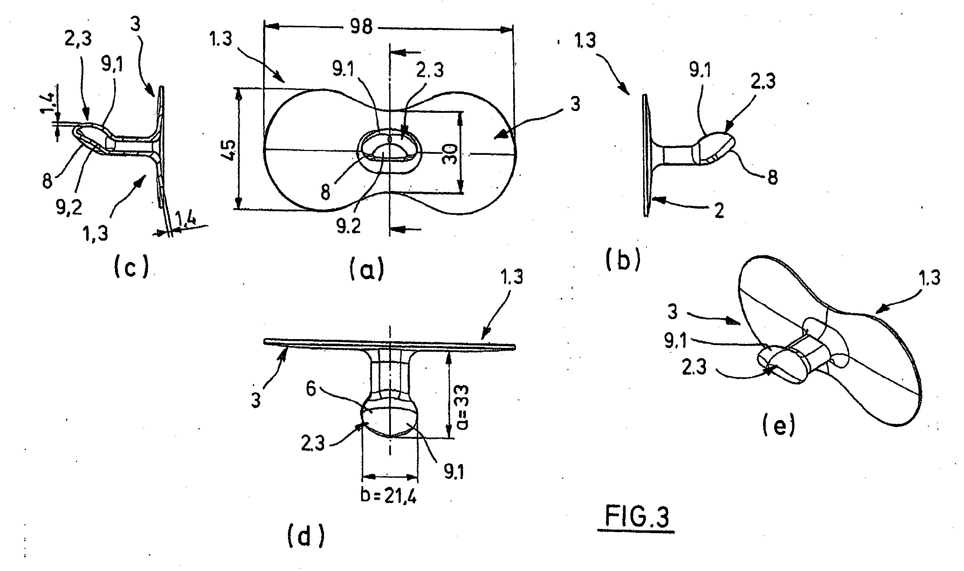

- Fig. 3a bis e: ein Saugutensil für einen Erwachsenen oder einen Träger mit einer großen Mundgröße in Vorderansicht (Fig. 3a), einer Ansicht von der linken Seite (Fig. 3b), einem Vertikalschnitt von rechts (Fig. 3c), einer Draufsicht (Fig. 3d) und in einer Perspektivansicht schräg von oben und von der rechten Seite (Fig. 3e).

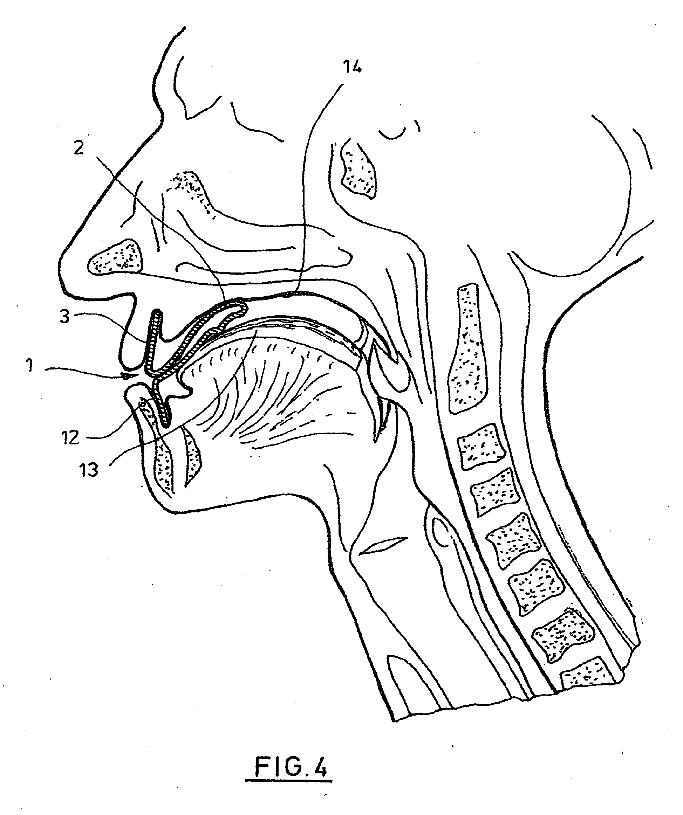

- Fig. 4: ein Saugutensil im Mund in einem Sagittalschnitt durch den Gesichts-Schädel eines Trägers;

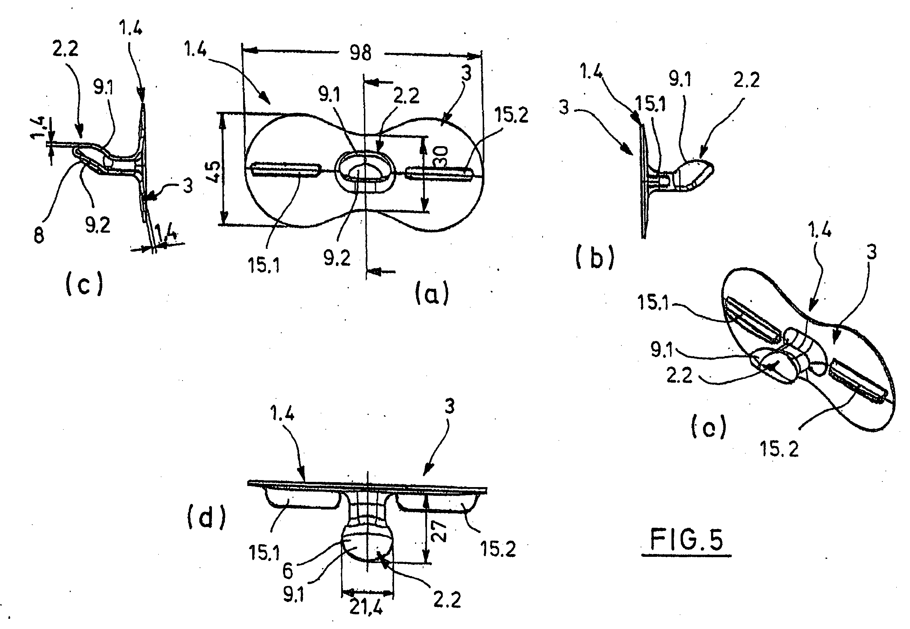

- Fig. 5a bis e: ein Saugutensil mit Aufbissleiste in Vorderansicht (Fig. 5a), einer Ansicht von der linken Seite (Fig. 5b), einem Vertikalschnitt von rechts (Fig. 5c), einer Draufsicht (Fig. 5d) und in einer Perspektivansicht schräg von oben und von der rechten Seite (Fig. 5e);

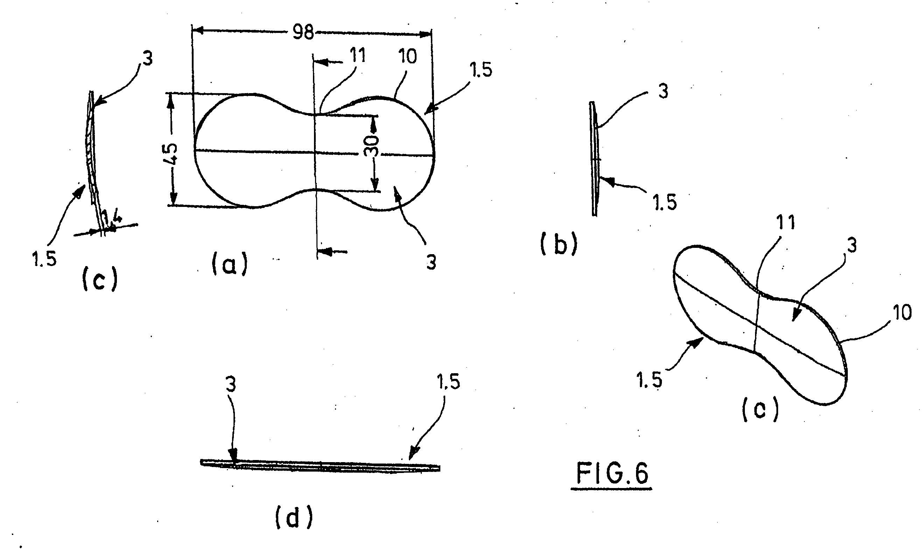

- Fig. 6a bis e: ein Saugutensil mit Mundvorhofschild ohne Saugernippel in Vorderansicht (Fig. 6a), einer Ansicht von der linken Seite (Fig. 6b), einem Vertikalschnitt von rechts (Fig. 6c), einer Draufsicht (Fig. 6d) und in einer Perspektivansicht schräg von oben und von der rechten Seite (Fig. 6e);

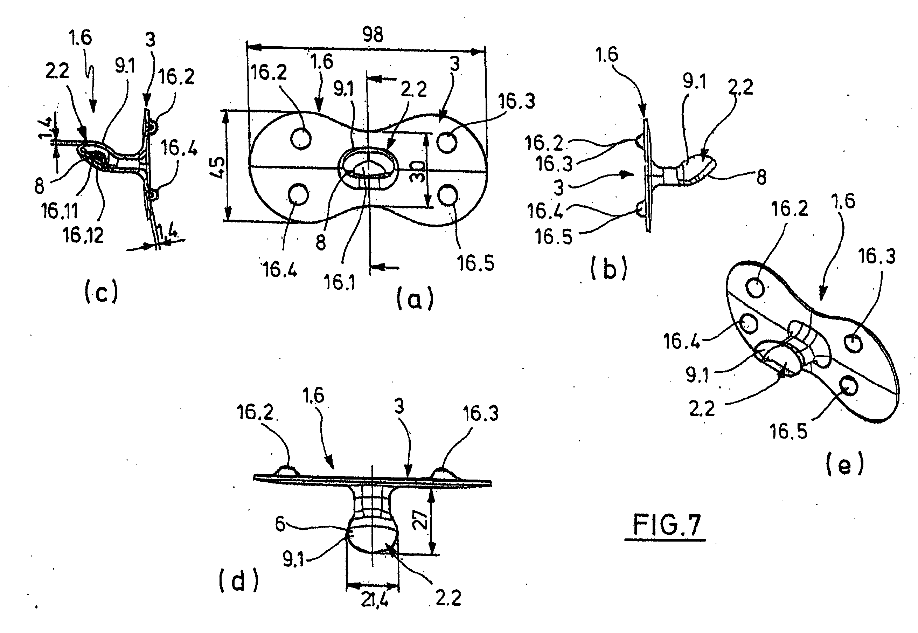

- Fig. 7a bis e: ein Saugutensil mit napfförmigen Vertiefungen in Vorderansicht (Fig. 7a), einer Ansicht von der linken Seite (Fig. 7b), einem Vertikalschnitt von rechts (Fig. 7c), einer Draufsicht (Fig. 7d) und in einer Perspektivansicht schräg von oben und von der rechten Seite (Fig. 7e);

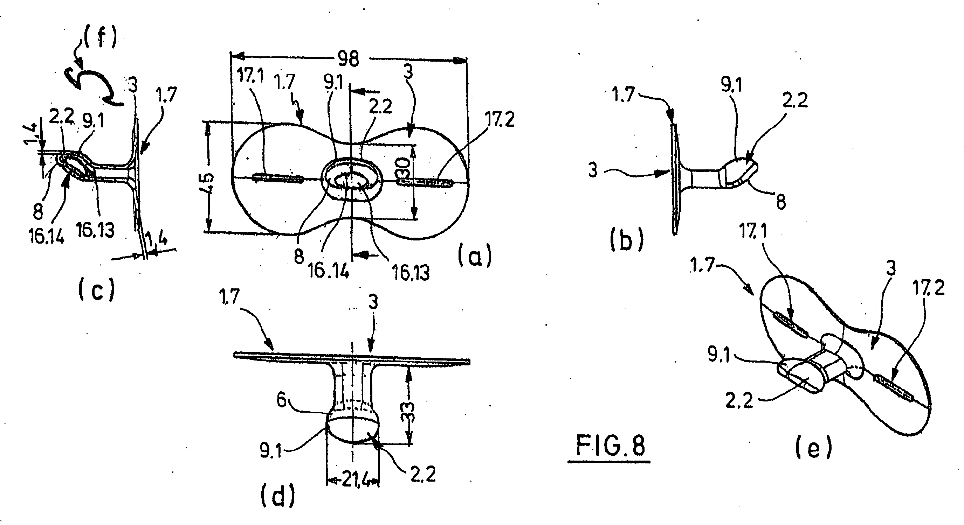

- Fig. 8a bis f: ein Saugutensil mit einer napfförmigen Vertiefung im Saugernippel und Schlitzen für Aufbissleisten im Mundvorhofschild in Vorderansicht (Fig. 8a), einer Ansicht von der linken Seite (Fig. 8b), einem Vertikalschnitt von rechts (Fig. 8c), einer Draufsicht (Fig. 8d) und in einer Perspektivansicht schräg von oben und von der rechten Seite (Fig. 8e) und das Profil der napfförmigen Vertiefung in einem versiegelten Schnitt durch die Sagitalebene (Fig. 8f);

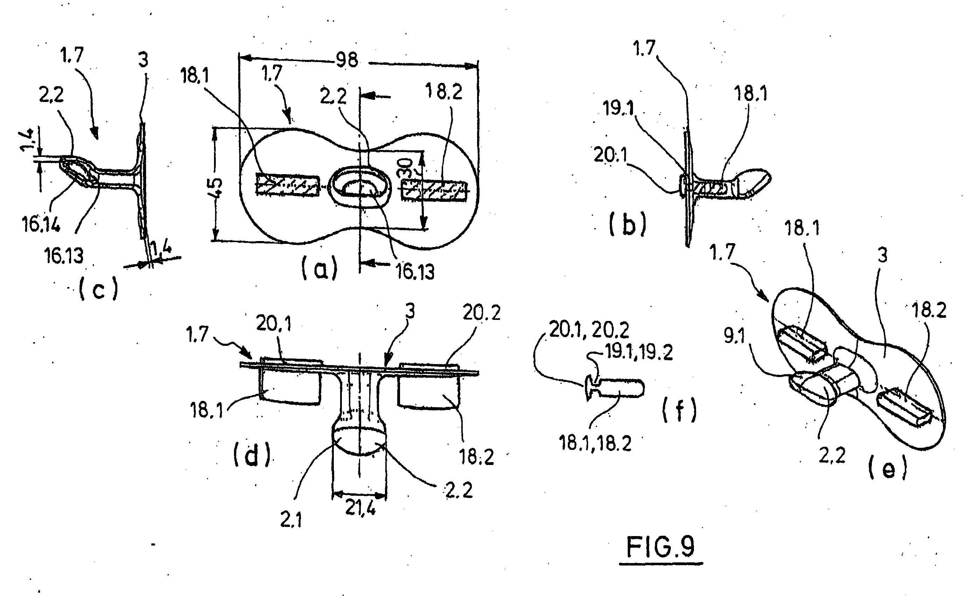

- Fig. 9a bis f: das Saugutensil von Fig. 7 mit verknüpften seitlichen Bissleisten in Vorderansicht (Fig. 9a), einer Ansicht von der linken Seite (Fig. 9b), einem Vertikalschnitt von rechts (Fig. 9c), einer Draufsicht (Fig. 9d) und in einer Perspektivansicht schräg von oben und von der rechten Seite (Fig. 9e) und eine Bissleiste in einer Seitenansicht (Fig. 9f);

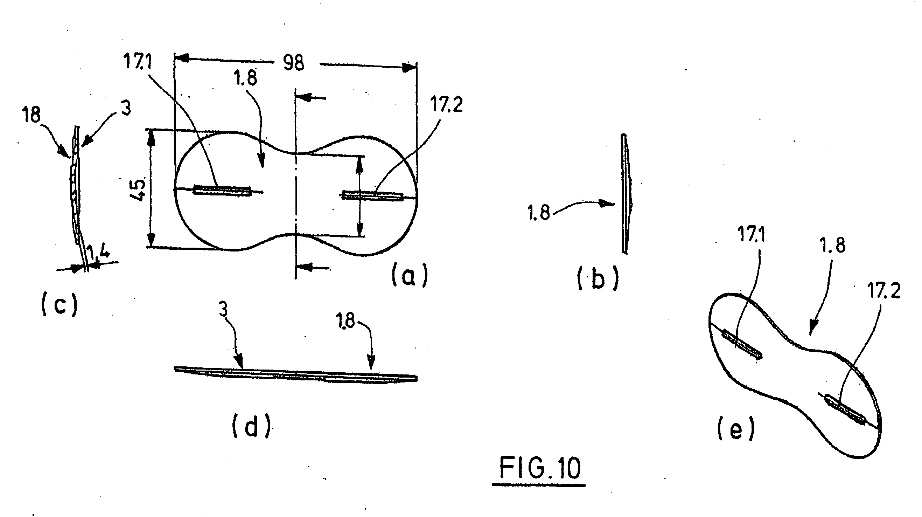

- Fig. 10a-e: ein Saugutensil mit Mundvorhofschild ohne Saugernippel mit Schlitzen für Bissleisten in Vorderansicht (Fig. 10a), einer Ansicht von der linken Seite (Fig. 10b), einem Vertikalschnitt von rechts (Fig. 10c), einer Draufsicht (Fig. 10d) und in einer Perspektivansicht schräg von oben und von der rechten Seite (Fig. 10e);

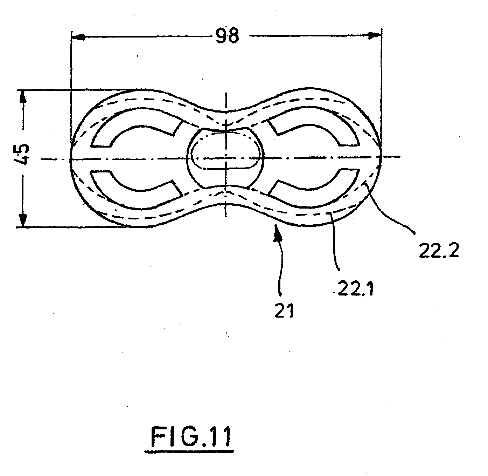

- Fig. 11: eine Schablone zum Zuschneiden eines Saugutensils in Vorderansicht;

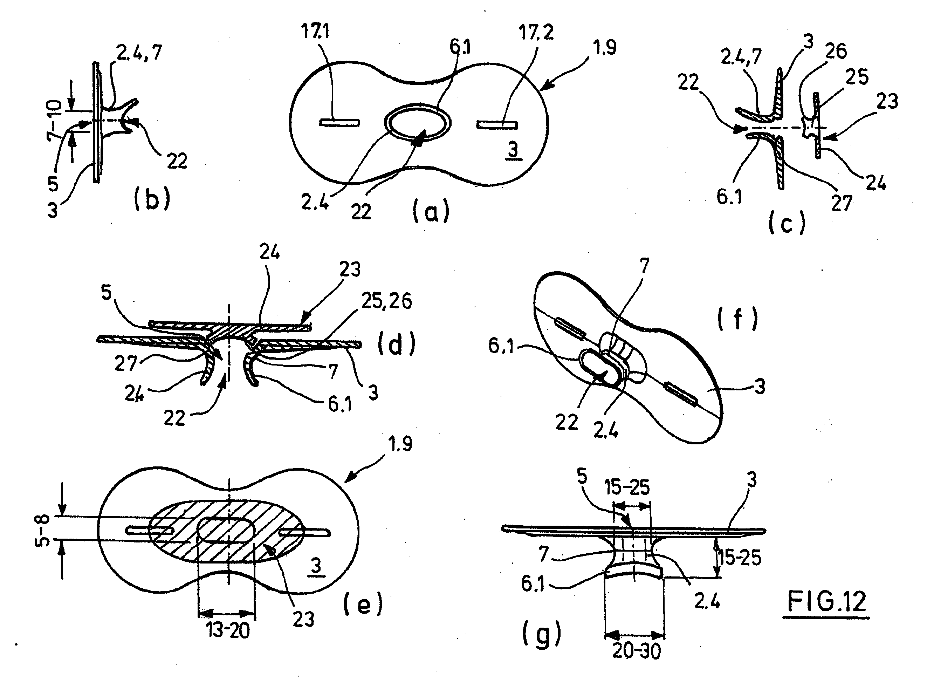

- Fig. 12a-g: ein Saugutensil mit einem Saugkörper mit Öffnungen im Mundvorhofschild und distaler Öffnung in Vorderansicht (Fig. 12a), in einer Ansicht von der linken Seite (Fig. 12b), mit Verschlussteil in einer Ansicht von der rechten Seite (Fig. 12c), mit Verschlussteil in einer Draufsicht (12d), mit Verschlussteil in einer Vorderansicht (Fig. 12e), ohne Verschlussteil in einer Perspektivansicht schräg von oben und von der rechten Seite (Fig. 12f) und ohne Verschlussteil in einer Draufsicht (Fig. 12g);

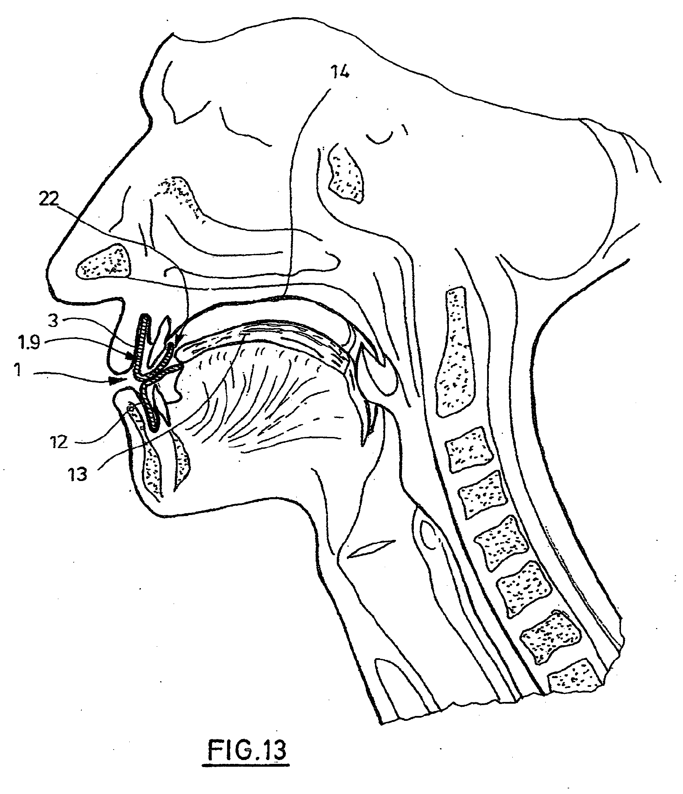

- Fig. 13: ein Saugutensil gemäß Fig. 12 im Mund in einem Sagittalschnitt durch den Gesichtsschädel eines Trägers;

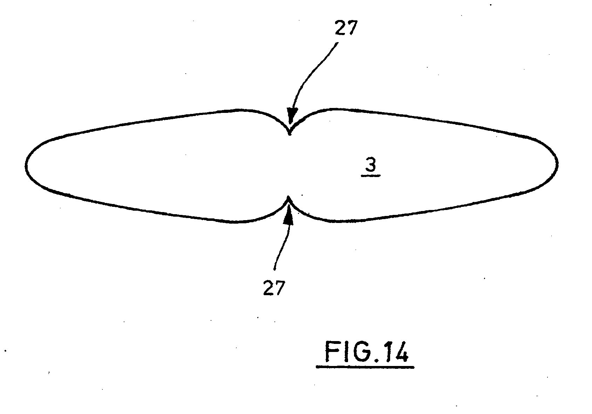

- Fig. 14: ein Mundvorhofschild mit einer Kontur mit V-förmiger Taille in einer Vorderansicht.

Bei der nachfolgenden Erläuterung sind entsprechende Teile verschiedener Ausführungsbeispiele mit denselben Bezugsziffern versehen. Gleich bezeichnete Teile, die unterschiedlich ausgebildet sind, sind durch einen den gleichen Bezugsziffern nachfolgenden Punkt und eine nachfolgende weitere Ziffer bezeichnet, wobei die nachfolgende Ziffer unterschiedlich ist.

Gemäß Fig. 1 hat ein erfindungsgemäßes Saugutensil 1.1 einen Saugernippel 2, der auf Basis einteilig mit einem Mundvorhofschild 3 verbunden ist.

Der Saugernippel 2.1 hat innen einen Hohlraum 4, der von der Außenseite des Mundvorhofschildes 3 aus durch eine Öffnung 5 des Mundvorhofschildes 3 hindurch zugänglich ist.

Der Saugkörper 2.1 weist einen Saugernippel 6 und einen einenends mit dem Saugernippel 6 und anderenends mit dem Mundvorhofschild 3 verbundenen Hals 7 auf. Der Saugkörper 2.1 ist im Bereich des Saugernippels 6 in einer Transversalebene (d.h. in einer zur Zeichenebene von Fig. 1d parallelen Ebene) breiter als der Hals 7.

Der Saugernippel 6 weist an der Unterseite eine Anschrägung 8 auf. An der Oberseite hat er eine konvexe Wölbung 9.1 und ist im Übergangsbereich zur Anschrägung 8 gerundet. Die Anschrägung 8 hat im Zentrum eine konkave Wölbung 9.2 bzw. eine Mulde. Der Hals 7 ist im Verbindungsbereich mit dem Saugernippel 6 und im Verbindungsbereich mit dem Mundvorhofschild 3 gerundet.

Der Saugkörper 2.1 hat insgesamt eine orthodontische Form.

Die Ausdehnung des Saugkörpers 2.1 in Richtung seiner Längsachse bzw. in sagittaler Richtung a beträgt 23 mm. Seine maximale Ausdehnung in transversaler Richtung b beträgt 21,4 mm. Seine Wandstärke c beträgt 1,4 mm.

Der Mundvorhofschild 3 weist eine katzenzungenförmige Kontur 10 auf. In lateraler Richtung hat der Mundvorhofschild 3 eine Ausdehnung d von 98 mm. Im Bereich der Taille 11 der Kontur 10 hat der Mundvorhofschild 3 eine minimale Ausdehnung e von 30 mm. Beidseitig neben der Taille 11 hat der Mundvorhofschild 3 eine maximale Ausdehnung f von jeweils 45 mm. Die Wandstärke g des Mundvorhofschildes 3 beträgt 1,4 mm. Ggf. ist die Wandstärke des Mundvorhofschildes unterschiedlich, z.B. zur Peripherie hin dünner auslaufend und zur Mitte hin (um die Saugernippelöffnung 5 herum) steifer, aber nicht unbedingt dicker (zur Stützung der Zahnfronten durch den Lippenschluss bzw. Verhinderung eines Zurücksinkens des Unterkiefers).

Das Saugutensil 1.1 ist einteilig aus Latex hergestellt. Stattdessen kann es aus Silikon oder aus einem thermoplastischen Elastomer hergestellt sein. Die Herstellung aus Latex erfolgt unter Einsatz eines Tauchverfahrens. Die Herstellung aus Silikon oder aus einem thermoplastischen Elastomer kann durch Spritzgießen erfolgen. Ferner sind Materialkombinationen möglich, wobei die verschiedenen Teile des Saugutensils aus verschiedenen Materialien bestehen können. Beispielsweise kann der Mundvorhofschild 3 aus thermoplastischem Elastomer und der Saugkörper 2.1 aus Silikon bestehen, wobei die Teile so gespritzt sein können, dass sie formschlüssig miteinander verbunden sind. Ferner kann ein mit dem Mundvorhofschild 3 integral verbundener Sicherheitsstreifen, der ebenfalls aus dem thermoplastischen Elastomer besteht, in den Saugkörper 2.1 aus Silikon eingebettet sein.

Vorstehende Erläuterungen gelten auch für die nachfolgenden Ausführungsbeispiele, soweit sie entsprechend ausgeführt sind. Nachfolgend werden besonders die Abweichungen der weiteren Ausführungsbeispiele von dem vorstehenden Ausführungsbeispiel erläutert.

Das Saugutensil 1.2 von Fig. 2 unterscheidet sich von dem Vorbeschriebenem dadurch, dass die Ausdehnung a des Saugkörpers 2.2 in Längsrichtung 27 mm beträgt.

Das Saugutensil 1.3 gemäß Fig. 3 unterscheidet sich von den Vorbeschriebenen dadurch, dass die Ausdehnung a des Saugkörpers 2.3 in seiner Längsrichtung 33 mm beträgt.

Gemäß Fig. 4 ist ein Saugutensil 1 im Mund eines Benutzers angeordnet. Der Mundvorhofschild 3 ist im Mundvorhof 12 angeordnet und der Saugkörper 2 ist zwischen Zunge 13 und Gaumen 14 angeordnet.

Der Saugreflex geht mit einer Anhebung und Spannung der Hinterzunge und einer Senkung und Spannung des Gaumensegels einher. Infolgedessen findet hinten ein aktiver Mundschluss am Oropharynx statt. Der weiche Gaumen verschließt sich mit dem hinteren Zungenrücken, sodass eine Nasenatmung möglich ist, nicht aber eine Mundatmung.

Ferner stellt sich ein reflexartiger Lippenschluss-Effekt ein. Hierbei senkt sich die Oberlippe und die Unterlippe hebt sich an. Oberlippe und Unterlippe umschließen bzw. umspannen den Mundvorhofschild 3. Infolgedessen ist ein aktiver vorderer Mundschluss am Vestibulum gegeben.

Der Mundschluss vorn und der Mundschluss hinten verhindern die Mundatmung einschließlich der geräuschhaften Vibration des Gaumensegels (Schnarchen).

Ein weiterer möglicher Begleiteffekt ist die Tonisierung bzw. das Training der labialen, lingualen und pharyngealen Muskeln. Diese Nachwirkung beruht auf einem Lerneffekt bei der Saugaktivität, so dass ggf. zumindest zeitweilig Atemgeräusche und/oder Mundatmung auch ohne den Einsatz des Saugers unterbleiben.

Ferner bewirkt das Tragen des Saugutensils 1 einen Vorschub des rückverlagerten Unterkiefers sowie einen Vorschub der Zunge und der Unterlippe. Dadurch wird der Abstand für die benötigten Okklusionen, d.h. der Kontakt Lippe zu Lippe, Unter- zu Oberkiefer und Zunge zu Gaumen begünstigt. Eine Mundatmung wird zugunsten der Nasenatmung eingestellt.

Fig. 5 zeigt ein Saugutensil 1.4, das im Unterschied zu dem Saugutensil 1.2 von Fig. 2 an der Innenseite des Mundvorhofschildes 3 beidseitig des Saugkörpers 2.2 jeweils eine Bissleiste 15.1, 15.2 trägt. Die Bissleisten 15.1, 15.2 sind fest mit dem Saugutensil 1.2 verbunden. In diesem Beispiel sind die Bissleisten 15.1, 15.2 einteilig mit dem Mundvorhofschild 3 und aus demselben Material wie der Mundvorhofschild 3 hergestellt. Die Bissleisten 15.1, 15.2 stehen etwa senkrecht vom Mundvorhofschild 3 vor. Die Bissleisten 15.1, 15.2 können auch länger oder kürzer ausgebildet sein. Ferner kann das Saugutensil 1.4 auch mit den Abmessungen der Saugutensile 1.1 oder 1.3 oder anderen Abmessungen ausgeführt sein.

Das Saugutensil 1.5 von Fig. 6 unterscheidet sich von dem Saugutensil 1.2 von Fig. 2 dadurch, dass es lediglich einen Mundvorhofschild 3 und keinen Saugkörper aufweist. Optional verjüngt sich der Mundvorhofschild 3 zu den Randbereichen hin ebenso wie der Mundvorhofschild 3 der Saugutensilien 1.1, 1.2 und 1.3. Das Saugutensil 1.5 kann auch mit den Abmessungen der Mundvorhofschilde 3 der Saugutensile 1.1 oder 1.3 oder anderen Abmessungen ausgeführt sein.

Fig. 7 zeigt ein Saugutensil 1.6, das im Unterschied zum Saugutensil 1.2 von Fig. 2 im Zentrum der Anschrägung 8 des Saugernippels 6 eine napfförmige Vertiefung 16.1 mit einer Öffnung 16.1,2 in der Anschrägung aufweist, die als Saugnapf wirkt. Die napfförmige Vertiefung 16.1 ist im Vergleich zur konkaven Wölbung 9.2 tiefer nach innen ausgewölbt, so dass nach Auspressen von Luft aus der napfförmigen Vertiefung 16.1 eine Anhaftung des Saugnapfes 16.1 am Zungenrücken möglich ist.

Ferner weist das Saugutensil 1.6 an der Innenseite des Mundvorhofschildes 3 weitere napfförmige Vertiefungen 16.2, 16.3, 16.4, 16.5 mit Öffnungen in der Innenseite des Mundvorhofschildes 3 auf, wobei die Paare der napfförmigen Vertiefungen 16.2, 16.3 und 16.4, 16.5 jeweils symmetrisch zur Sagittalebene und die Paare der napfförmigen Vertiefungen 16.2, 16.4 und 16.3, 16.5 jeweils symmetrisch zur Transversalebene angeordnet sind. Die Saugnäpfe 16.2 bis 16.5 können am Zahnfleisch anhaften und verstärken damit den dichten Abschluss des Mundschildes. Die napfförmige Vertiefung 16.1 verleitet den Träger auch zum Spielen mit der Zungenspitze an dem Saugernippel 6.

Der Saugernippel 6 kann an der Oberseite 9.1 mit einer napfförmigen Vertiefung versehen sein, die ein Anhaften an dem Gaumen bewirkt.

Fig. 8 zeigt ein Saugutensil 1.7, das im Unterschied zum Saugutensil 1.6 von Fig. 7 in der Anschrägung 8 des Saugernippels 6 eine stärker ausgeprägte napfförmige Vertiefung 16.1,2 aufweist. Die napfförmige Vertiefung 16.1,2 ist im Sagitalschnitt wie ein Omega ausgebildet, so dass sie im Bereich ihrer Öffnung 16.1.4 in der Anschrägung 8 einen geringeren Querschnitt aufweist als einem weiter innen im Saugernippel 6 angeordneten Bereich.

Ferner unterscheidet sich das Saugutensil 1.7 von dem Saugutensil 1.6 dadurch, dass der Mundvorhofschild 3 beidseitig des Saugkörpers 2.2 Schlitze 17.1, 17.2 zum Einknüpfen von Bissleisten aufweist. Der Benutzer kann also das Saugutensil 1.7 wahlweise ohne oder mit Bissleisten benutzen. Darüber hinaus fehlen bei dem Mundvorhofschild 3 des Saugutensils 1.7 die napfförmigen Vertiefungen 16.2 bis 16.5 sowie an der Oberseite 9.1 des Saugernippels 6 (nicht abgebildet).. Eine alternative Ausgestaltung des Saugutensils 1.7, die nicht abgebildet ist, weist die napfförmigen Vertiefungen 16.2 bis 16.5 sowie an der Oberseite 9.1 des Saugernippels 6 auf.

Gemäß Fig. 9 ist das Saugutensil 1.7 von Fig. 8 mit Bissleisten 18.1, 18.2 ausgestattet. Jede Bissleiste 18.1, 18.2 hat an einer Stirnseite einen Steg 19.1, 19.2, der endseitig eine Halteplatte 20.1, 20.2 trägt. Statt des Steges 19.1, 19.2 können auch mehrere Zapfen vorhanden sein, an denen endseitig die Halteplatte angeordnet ist oder die jeweils mit einer eigenen Halteplatte versehen sind.

Die Halteplatten 20.1, 20.2 sind durch die Schlitze 17.1, 17.2 des Mundvorhofschildes 3 hindurch geführt, so dass die Stege 19.1, 19.2 in den Schlitzen 17.1, 17.2 angeordnet sind, die Halteplatte 20.1, 20.2 auf der Rückseite und die Bissleisten 18.1, 18.2 auf der Vorderseite des Mundvorhofschildes 3 abgestützt sind.

Die Bissleisten 18.1, 18.2 können Ein- und/oder Aufbissleisten sein. Eine Einbissleiste kann durch hineinbeißen an die Form der Zähne des Benutzers angepasst werden. Gegebenenfalls ist die Bissleiste 18.1, 18.2 so beschaffen, dass sie ein Einbeißen nach zeitweiliger Temperaturerhöhung (z. B. im siedenden Wasserbad) ermöglicht und ihre Form nach dem Erkalten (im kühlen Wasserbad) beibehält.

Der Mundvorhofschild 3 bzw. das Saugutensil 1.8 von Fig. 10 unterscheidet sich von dem Mundvorhofschild 3 bzw. Saugutensil 1.5 von Fig. 6 dadurch, dass es beidseitig der Sagitalebene mit in der Transversalebene erstreckten Schlitzen 17.1, 17.2 zum Einknüpfen einer Bissleiste versehen ist. Je nach Bedarf kann der Benutzer das Mundvorhofschild 3 ohne Bissleisten oder mit Bissleisten benutzen. Die in Fig. 9 gezeigten Bissleisten 18.1, 18.2 können zum Einsatz kommen.

Fig. 11 zeigt eine Schablone 21, die eine katzenzungenförmige Randkontur aufweist. Die Schablone 21 weist ferner im Abstand von der Randkontur Markierungslinien 22.1, 22.2 unterschiedlicher Form auf.

Die Schablone 21 kann auf einem Mundvorhofschild 3 aufgesetzt werden, der eine ursprünglich beliebige Kontur aufweist. Der äußere Rand der Schablone 21 oder die Markierungslinien 23.1, 23.2 sind zum Zuschneiden des Mundvorhofschildes 3 mittels einer Schere nutzbar.

Das Saugutensil 1.9 von Fig. 12 unterscheidet sich von dem Saugutensil 1.7 von Fig. 8 dadurch, dass der Saugernippel 6.1 nicht den distalen Teil des Saugernippels 6 hat. Dort hat der Saugkörper 2.4 eine distale Öffnung 22. Ferner weist der Saugkörper 2.4 im Mundvorhofschild 3 eine Öffnung 5 auf.

Der Saugkörper 2.4 erweitert sich angrenzend an seinen Hals 7 kelchförmig zur distalen Öffnung 22 hin. Im horizontalen Schnitt (in der Transversalebene bei Anordnung des Saugutensils im Mund des Trägers) nimmt die Aufweitung des Querschnittes angrenzend an den Hals 7 zunächst stärker und nimmt die Aufweitung in der Nähe der distalen Öffnung 22 schwächer zu (Fig. 12g). Im vertikalen Schnitt (in der Sagittalebene) erweitert sich der Saugkörper 2.4 vom Hals 7 ausgehend zur distalen Öffnung 22 annähernd gleichförmig oder asymmetrisch, wobei vorzugsweise oben länger und stärker gewölbt ist als unten (Fig. 12b).

In die Öffnung 5 im Mundvorhofschild 3 ist ein Verschlussteil 23 einknöpfbar. Das Verschlussteil 23 hat eine Verschlussplatte 24 und einen Druckknopf 25, der am distalen Ende eine radiale Erweiterung bzw. Wulst 26 aufweist. Das Verschlussteil 23 ist vorzugsweise aus demselben Material wie das Saugutensil 1.9 gefertigt.

Das Verschlussteil 23 ist in die Öffnung 5 einknöpfbar. Hierbei wird der Druckknopf 25 in den Mundvorhofschild 3 und/oder Saugkörper 2.4 eingepresst, wodurch ein fester Sitz des Verschlussteiles 23 im Saugkörper 2.4 sichergestellt wird. Gemäß Fig. 12c kann das Saugutensil 1.9 im Mundvorhofschild 3 und/oder Saugkörper 2.4 neben der Öffnung 5 eine umlaufende Nut oder Rille oder andere Vertiefung 27 aufweisen, in die die radiale Erweiterung 26 des Druckknopfes 25 einknöpfbar ist.

Das Verschlussteil 23 besteht vorzugsweise aus Naturkautschuk und/oder Silikon und/oder Elastomer und/oder einem thermoplastischen Elastomer.

Das Saugutensil 1.9 hat Schlitze 17.1, 17.2 im Mundvorhofschild 3 und kann wie das Saugutensil 1.7 von Fig. 9 mit Bissleisten 18.1, 18.2 versehen werden.

Vorzugsweise bestehen die Bissleisten aus einem Material, das an die Form der Zähen anpassbar ist.

Fig. 13 zeigt das Saugutensil 1.9 im Mund eines Benutzers. Die Spitze der Zunge 13 greift in die distale Öffnung 22 des Saugkörpers 2.4 ein.

Gemäß Fig. 14 weist ein Mundvorhofschild 3 vorzugsweise eine langgestreckte Kontur auf, die sich zu den beiden Enden hin verjüngt. In der Mitte weist der Mundvorhofschild 3 eine Taille auf, die durch beidseitige, V-förmige Aussparungen 27 gebildet ist. Dergestalt kann der Mundvorhofschild 3 bei sämtlichen Ausführungsbeispielen ausgestaltet sein.

## Patentansprüche

1. Saugutensil zum Verhindern von Gewohnheiten wie Schnarchen, Mundatmung, Lutschen, Knirschen und Pressen der Zähne und zum Trainieren oder Therapieren oraler Funktionen umfassend einen zumindest teilweise aus einem flexiblen Flachmaterial bestehenden, sich an die Oberfläche der Zähne und des Zahnfleisches anpassenden Mundvorhofschild (3) und einen von der Innenseite des Mundvorhofschildes (3) vorstehenden und an seiner Basis mit dem Mundvorhofschild (3) verbundenen weichelastischen Saugkörper (2), **dadurch gekennzeichnet, dass** der Mundvorhofschild so lang ist, dass er seitlich zumindest bis zu den Prämolaren reicht.

2. Saugutensil nach Anspruch 1, bei dem der Saugkörper (2) einen durch eine Öffnung (5) in der Außenseite des Mundvorhofschildes zugänglichen Hohlraum (4) aufweist.

3. Saugutensil nach Anspruch 2, bei dem der Saugkörper (2) einen Saugernippel (6) und einen einenends mit dem Saugernippel (6) und anderenends mit dem Mundvorhofschild (3) verbundenen Hals (7) aufweist.

4. Saugutensil nach einem der Ansprüche 1 bis 3, bei dem der Saugkörper (2) eine orthodontische Form hat.

5. Saugutensil nach einem der Ansprüche 1 bis 4, bei dem der Saugkörper (2) und/oder der Mundvorhofschild (3) mindestens eine napfförmige Vertiefung (16) aufweist.

6. Saugutensil nach Anspruch 5, bei dem der Saugkörper (2) die napfförmige Vertiefung (16) in einer Fläche aufweist, an der beim Gebrauch die Zunge anliegt und/oder in einer Fläche, an der beim Gebrauch der Gaumen anliegt, und/oder bei dem der Mundvorhofschild (3) die napfförmige Vertiefung (16) in einer Fläche aufweist, die beim Gebrauch dem Zahnfleisch oder der Wangenschleimhaut zugewandt ist.

7. Saugutensil nach einem der Ansprüche 1 bis 6, bei dem der Saugkörper (2) eine distale Öffnung (22) aufweist.

8. Saugutensil nach Anspruch 7, bei dem sich der Saugkörper (2) zur distalen Öffnung (22) hin erweitert.

9. Saugutensil nach einem der Ansprüche 7 und 8 sowie 2, bei dem die Öffnung (5) im Mundvorhofschild (3) mittels eines lösbar am Saugerutensil (1) gehaltenen Verschlussteils (23) verschlossen ist.

10. Saugutensil nach einem der Ansprüche 1 bis 9, bei dem der distal geschlossene Saugkörper (2) in Richtung seiner Längsachse eine Ausdehnung von 20 bis 35 mm aufweist, und bei dem der Saugkörper (2) mit distaler Öffnung (22) in Richtung seiner Längsachse eine Ausdehnung von 15-25 mm aufweist.

11. Saugutensil nach einem der Ansprüche 1 bis 10, bei dem der Mundvorhofschild (3) eine an die Anatomie des Mundvorhofs angepasste Kontur (10) aufweist und/oder bei dem der Mundvorhofschild (3) eine langgestreckte Kontur aufweist und/oder bei dem die Kontur (10) des Mundvorhofschildes (3) an den gingivalen Rändern im Nachbarbereich zu den Lippenbändchen im Ober- und Unterkiefer jeweils eine Taille (11) hat und/oder bei dem der Mundvorhofschild (3) eine in Umfangsrichtung im gesamten Verlauf gerundete Kontur (10) hat.

12. Saugutensil nach Anspruch 11, bei dem der Mundvorhofschild (3) lateral eine maximale Ausdehnung von 80 bis 140 mm und/oder an der Taille (11) eine minimale Ausdehnung von 15 bis 35 mm und/oder beidseitig neben der Taille (11) eine maximale Ausdehnung von 25 bis 50 mm aufweist.

13. Saugutensil nach einem der Ansprüche 1 bis 12, bei dem der Mundvorhofschild (3) mindestens eine vom Rand des Mundvorhofschildes (3) beabstandete Markierungslinie mit einem der variierenden Anatomie des Mundvorhofs und/oder der Kontur des Mundvorhofschildes angepassten Verlauf hat.

14. Saugutensil nach einem der Ansprüche 1 bis 13 mit mindestens einer von der Innenseite des Mundvorhofschildes (3) vorstehenden Bissleiste (15.1, 15.2).

15. Saugutensil nach Anspruch 14 mit jeweils einer im Bereich der Frontzähne und/oder einer im Seitenzahnbereich jeweils vom Mundvorhofschild vorstehenden Bissleiste (15.1, 15.2) und/oder bei dem der Saugkörper (2) und/oder die Bissleiste (15.1, 15.2) durch Befestigungsmittel oder einteilig mit dem Mundvorhofschild (3) verbunden ist.

## Claims

1. A teat device for preventing habits, such as snoring, mouth breathing, sucking, grinding and clenching the teeth, and for training or treating oral functions, comprising an oral vestibular shield (3) that at least partially consists of a flexible flat material, adapting to the surface of the teeth and the gingiva, and a soft elastic suction body (2) projecting from the inside of the oral vestibular shield (3) and connected at the base thereof to the oral vestibular shield (3), **characterized in that** the oral vestibular shield is so long such that it extends laterally at least to the premolars.

2. The teat device according to claim 1, wherein the suction body (2) has a cavity (4) that is accessible through an opening (5) in the outside of the oral vestibular shield.

3. The teat device according to claim 2, wherein the suction body (2) has a teat (6) and a neck (7) connected at one end to the teat (6) and at the other end to the oral vestibular shield (3).

4. The teat device according to one of claims 1 to 3, wherein the suction body (2) has an orthodontic shape.

5. The teat device according to one of claims 1 to 4, wherein the suction body (2) and/or the oral vestibular shield (3) has at least one cup-shaped recess (16).

6. The teat device according to claim 5, wherein the suction body (2) has the cup-shaped recess (16) in a surface against which the tongue rests during use, and/or in a surface against which the palate rests during use, and/or wherein the oral vestibular shield (3) has the cup-shaped recess (16) in a surface that faces the gingiva or buccal mucosa during use.

7. The teat device according to one of claims 1 to 6, wherein the suction body (2) has a distal opening (22).

8. The teat device according to claim 7, wherein the suction body (2) expands toward the distal opening (22).

9. The teat device according to one of claims 7 and 8 as well as 2, wherein the opening (5) in the oral vestibular shield (3) is sealed by means of a sealing part (23) that is releasably held on the teat device (1).

10. The teat device according to one of claims 1 to 9, wherein the distally closed suction body (2) has an expansion of 20 to 35 mm in the direction of its longitudinal axis, and wherein the suction body (2) with a distal opening (22) in the direction of its longitudinal axis has an expansion of 15-25 mm.

11. The teat device according to one of claims 1 to 10, wherein the oral vestibular shield (3) has a contour (10) that is adapted to the anatomy of the oral vestibule and/or wherein the oral vestibular shield (3) has an elongated contour, and/or wherein the contour (10) of the oral vestibular shield (3) has a tapering (11) at the gingival margins proximate to the labial frenulum in the maxilla and mandible and/or wherein the oral vestibular shield (3) has a rounded contour (10) over the entire course in the peripheral direction thereof.

12. The teat device according to claim 11, wherein the oral vestibular shield (3) has a lateral maximum expansion of 80 to 140 mm, and/or a minimum expansion of 15 to 35 mm at the tapering (11), and/or a maximum expansion of 25 to 50 mm at both sides beside the tapering (11).

13. The teat device according to one of claims 1 to 12, wherein the oral vestibular shield (3) has at least one marking line at a distance from the edge of the oral vestibular shield (3), with an outline adapted to the varying anatomy of the oral vestibule and/or the contour of the oral vestibular shield.

14. The teat device according to one of claims 1 to 13 having at least one bite splint (15.1, 15.2) projecting from the inside of the oral vestibular shield (3).

15. The teat device according to claim 14, having a bite splint (15.1, 15.2) that in each case projects from the oral vestibular shield in the region of the anterior teeth and/or in the lateral tooth region, and/or wherein the suction body (2) and/or the bite splint (15.1, 15.2) is connected to the oral vestibular shield (3) by means of fasteners or as a single piece.

## Revendications

1. Appareil d'aspiration pour empêcher des habitudes comme le ronflement, la respiration buccale, le suçage, le grincement et la pression des dents et pour l'entraînement ou la thérapie de fonctions orales, comprenant un écran vestibulaire buccal (3) constitué au moins partiellement d'un matériau plat flexible s'adaptant à la surface des dents et des gencives et un corps d'aspiration (2) élastique souple protubérant du côté intérieur de l'écran vestibulaire buccal (3) et relié au niveau de sa base à l'écran vestibulaire buccal (3), **caractérisé en ce que** la longueur de l'écran vestibulaire buccal est telle qu'il atteigne latéralement au moins les prémolaires.

2. Appareil d'aspiration selon la revendication 1, dans lequel le corps d'aspiration (2) présente une cavité (4) accessible par un orifice (5) dans la face extérieure de l'écran vestibulaire buccal.

3. Appareil d'aspiration selon la revendication 2, dans lequel le corps d'aspiration (2) présente un raccord d'aspiration (6) et un col (7) relié sur un côté au raccord d'aspiration (6) et sur l'autre côté à l'écran vestibulaire buccal (3).

4. Appareil d'aspiration selon l'une quelconque des revendications 1 à 3, dans lequel le corps d'aspiration (2) a une forme orthodontique.

5. Appareil d'aspiration selon l'une quelconque des revendications 1 à 4, dans lequel le corps d'aspiration (2) et/ou l'écran vestibulaire buccal (3) présente(nt) au moins un évidement (16) en forme de bol.

6. Appareil d'aspiration selon la revendication 5, dans lequel le corps d'aspiration (2) présente l'évidement (16) en forme de bol dans une surface sur laquelle la langue est collée en cours d'utilisation et/ou dans une surface sur laquelle le palais est collé en cours d'utilisation et/ou dans lequel l'écran vestibulaire buccal (3) présente l'évidement (16) en forme de bol dans une surface qui est orientée en cours d'utilisation vers les gencives ou les muqueuses de la joue.

7. Appareil d'aspiration selon l'une quelconque des revendications 1 à 6, dans lequel le corps d'aspiration (2) présente un orifice distal (22).

8. Appareil d'aspiration selon la revendication 7, dans lequel le corps d'aspiration (2) s'élargit vers l'orifice distal (22).

9. Appareil d'aspiration selon l'une quelconque des revendications 7 et 8 ainsi que 2, dans lequel l'orifice (5) dans l'écran vestibulaire buccal (3) est obturé au moyen d'une pièce de fermeture (23) fixée de manière amovible à l'appareil d'aspiration (1).

10. Appareil d'aspiration selon l'une quelconque des revendications 1 à 9, dans lequel le corps d'aspiration (2) fermé distalement présente dans le sens de son axe longitudinal une extension de 20 à 35 mm, et dans lequel le corps d'aspiration (2) avec orifice distal (22) présente dans le sens de son axe longitudinal une extension de 15-25 mm.

11. Appareil d'aspiration selon l'une quelconque des revendications 1 à 10, dans lequel l'écran vestibulaire buccal (3) présente un contour (10) adapté à l'anatomie du vestibule buccal et/ou dans lequel l'écran vestibulaire buccal (3) présente un contour allongé et/ou dans lequel le contour (10) de l'écran vestibulaire buccal (3) a sur les bords gingivaux dans la zone avoisinante des freins labiaux dans la mâchoire supérieure et inférieure respectivement une taille (11) et/ou dans lequel l'écran vestibulaire buccal (3) a un contour (10) arrondi dans le sens circonférentiel sur tout le contour.

12. Appareil d'aspiration selon la revendication 11, dans lequel l'écran vestibulaire buccal (3) présente latéralement une extension maximale de 80 à 140 mm et/ou à la taille (11) une extension minimale de 15 à 35 mm et/ou sur les deux côtés près de la taille (11) une extension maximale de 25 à 50 mm.

13. Appareil d'aspiration selon l'une quelconque des revendications 1 à 12, dans lequel l'écran vestibulaire buccal (3) présente au moins une ligne de marquage espacée du bord de l'écran vestibulaire buccal (3) avec un tracé adapté à l'anatomie variable du vestibule buccal et/ou au contour de l'écran vestibulaire buccal.

14. Appareil d'aspiration selon l'une quelconque des revendications 1 à 13 avec au moins une gouttière (15.1, 15.2) protubérant du côté intérieur de l'écran vestibulaire buccal (3).

15. Appareil d'aspiration selon la revendication 14 avec une gouttière (15.1, 15.2) protubérant respectivement au niveau des dents de devant et/ou au niveau des dents latérales de l'écran vestibulaire buccal et/ou dans lequel le corps d'aspiration (2) et/ou la gouttière (15.1, 15.2) est relié(e) par des moyens de fixation à l'écran vestibulaire buccal (3) ou fait corps avec ce dernier.
